# FASCICULE DE BREVET EUROPEEN

(11) **EP 2 439 734 B1**
(45) Date de publication et mention de la délivrance du brevet: **19.11.2014**
(21) Numéro de dépôt: 11182672.3
(22) Date de dépôt: 26.09.2011
(51) Int. Cl.: G10K 11/172, A61F 11/08

(54) **Filtre acoustique à insérer dans un bouchon d'oreille**
Akustischer Filter zum Einsetzen in einen Ohrstöpsel
Acoustic filter to be inserted in an earplug

(30) Priorité: 11.10.2010 FR 1004033
(43) Date de publication de la demande: 11.04.2012
(73) Titulaire: Roussel, Pascal, 76160 Saint Martin du Vivier (FR)
(72) Inventeur: Roussel, Pascal, 76160 Saint Martin du Vivier (FR)
(74) Mandataire: Novagraaf Technologies

(56) Documents cités:
- FR-A1- 2 733 344
- FR-A1- 2 925 291
- GB-A- 191 404 821
- US-A1- 2003 159 878
- US-A1- 2006 042 867

## Description

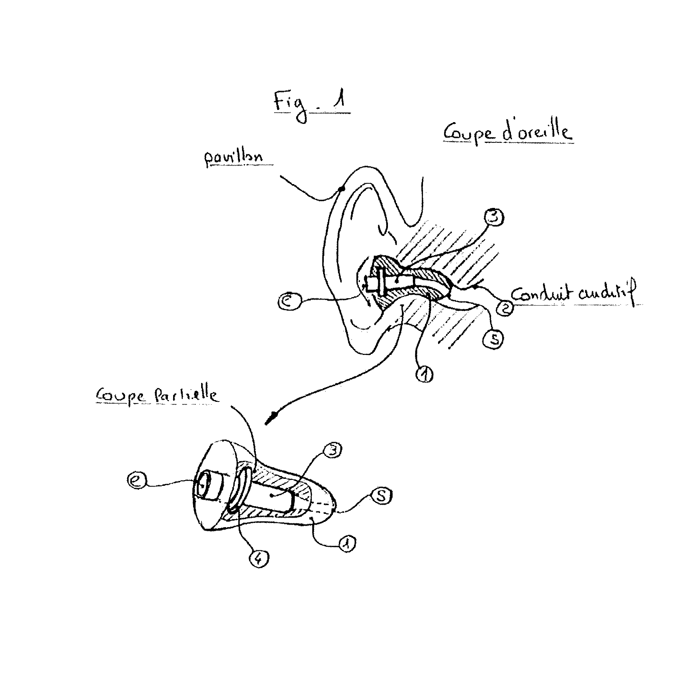

La présente invention concerne un dispositif de protection antibruit permettant al perception des sons atténués tout en minimisant, avec une pente contrôlée de 2 dB/octave, la déformation fréquentielle habituellement inhérente au port de protections antibruit. La courbe de réponse dudit dispositif et dite plate ou linéaire. Cette Invention est à insérer dans un bouchon d'oreille standard ou sur mesure (1. Fig1) de type mousse, acrylique ou silicone épousant parfaitement les formes du conduit auditif (2. Fig1) et formant une parfaite étanchéité au son. Le bouchon d'oreille sera ensuite percé de part et d'autre afin d'y insérer la présente invention (3.Fig1).

On connaît aujourd'hui différents systèmes de protection antibruit permettant la protection aux nuisances sonores du particulier ou du salarié exposé. Ces protections atténuent la perception des sons d'une façon différente entre les fréquences graves, mediums et aigues.
- Les casques antibruit dont l'atténuation sur les fréquences graves et forte ce qui est dû en partie à l'appui mécanique du casque sur la mastoïde (os dernière l'oreille en liaison avec la cochlée organe de l'audition) permettant le blocage des vibrations basses fréquences.
- Les mousses antibruit ou arceau à insérer dan le conduit auditif dont l'atténuation sur les fréquences aigues est forte ce qui est dû en partie à la perte d'insertion ou effet etymotique consécutif à la modification de l'amplification naturelle du conduit auditif sur les 3500 à 4000 Hz.
- Il existe aussi des protections moulées sur mesure avec un système de filtre permettant de laisser passer en partie les sons mediums ou fréquences du fondamental laryngé entre 250 à 500 Hz afin d'avoir une meilleure réponse de perception sur les fréquences de la voix humaine

Le document FR-A-2 925 291 présente un bouchon d'oreille destiné à la protection acoustique et comprenant une pièce cylindrique formant l'entrée acoustique, un filtre acoustique et une pièce cylindrique pourvue d'un canal de forme tronconique formant la sortie acoustique.

Pour les utilisateurs de ces antibruits la perception des sons est fortement déformée et peut poser un réel problème pour certaines applications, circonstances ou métiers où il est nécessaire de se protéger du bruit mais en conservant une bonne proportionnalité sur l'ensemble du spectre auditif sonore dans les graves, médiums et aigues. C'est le cas dans le monde de la musique et du son, des services dans les aéroports et généralement les transports, les particuliers au cinéma, au concert, pour les personnes atteintes d'hyperacoustie, etc...

La présente invention proposera un dispositif permettant de palier à ces problèmes en apportant une protection auditive d'atténuation variant environ de 10 à 25 dB avec une courbe de réponse régulée d'une pente de 2 dB/octave. De ce fait avec une atténuation de départ sur les fréquences graves à 125 Hz de 8 dB on obtiendra environ 10 dB à 250 Hz, environ 12 dB à 500 Hz pour finir dans le spectre sonore sans différence sensible de d'autre part une protection légèrement plus marquée sur les fréquences aigues utile pour la sauvegarde de la bonne audition de l'utilisateur exposé.

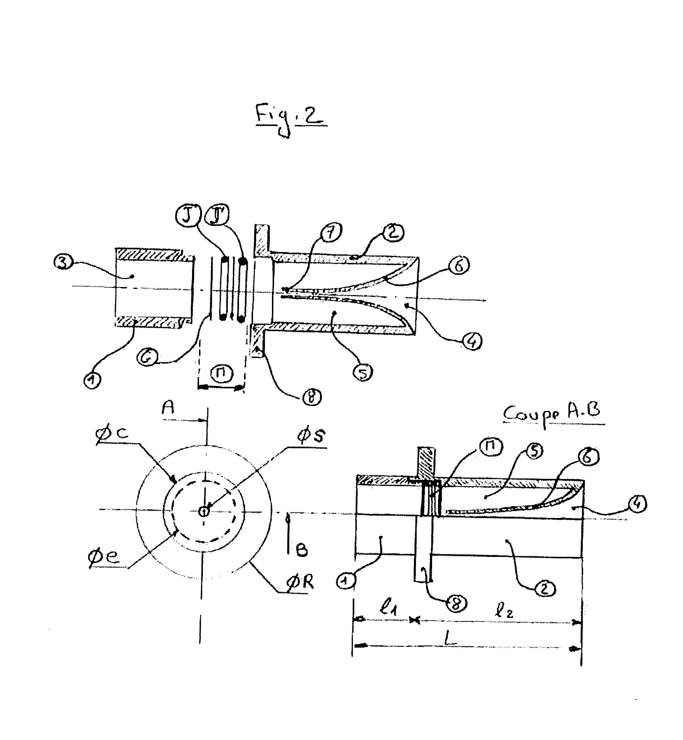

L'invention consiste en un ensemble de pièces assemblées dans un ordre et un sens de fonctionnement pour former au final une pièce cylindrique (3.Fig1) de 5 à 8 mm de diamètre (ØC. Fig2) et de 10 à 20 mm de long (L.Fig2), les deux pièces formant le cylindre seront réalisées par exemple par injection plastique. Le corps de ce cylindre étant composé d'une première pièce appelée « entrée acoustique » (1.Fig2) de 2 à 5 mm de long (M.Fig2) de 0,2 à 1 mm d'épaisseur et ayant une porosité de 7 à 100 µm protégé par une grille métallique inox (G.Fig2) de 0,25 mm d'épaisseur. Cette ensemble de pièces est appelé « empilement acoustique » (M.Fig2). Assemblé à cette première pièce « entrée acoustique » par collage ou clipsage vient s'ajuster une pièce appelée « sortie acoustique » (2.Fig2) de 8 à 15 mm de long (12.Fig2). Afin d'être certain de l'étanchéité sur le pourtour de la (ou les) membrane poreuse, un ou deux joints toriques (J-J'.Fig2) de 50 ou 70 sh viennent jointer l'assemblage. La sortie du filtre est composée d'un « cône acoustique » (6.Fig2) de forme hyperbolique de type f(x)=1/x². La pointe du cône étant ouverte (7.Fig2) et orientée vers la membrane en son centre et formant un diamètre d'entrée du « cône acoustique » (Øs.Fig2) de 0,2 à 1 mm. La pièce de « sortie acoustique » étant creuse, le volume intérieur résultant du volume total de la pièce de sorte moins le volume du « cône acoustique » compose le « résonateur acoustique » (5.Fig2). Le sens de fonctionnement (3.Fig2), passe par la (ou les) membrane poreuse (M.Fig2) efficace à l'atténuation des fréquences basses et poreuse (M.Fig2) efficace à l'atténuation des fréquences basses et médiums-basses, le son arrive dans le résonateur (5.Fig2) pour la sélection des fréquences médiums-hautes, puis ressort par le cône acoustique hyperbolique (6.Fig2) favorable à l'amplification des fréquences aigues afin de compenser la perte d'insertion [29-31]. Le contrôle des différentes atténuations de 10 à 25 dB suivant le résultat à obtenir se fera par modification des différentes cotes de longueur, de diamètre et d'épaisseur des pièces de l'invention ainsi que l'indice de porosité de ou des membranes dans « l'empilement acoustique » (M.Fig2).

Afin de maintenir le filtre dans le bouchon d'oreille il est prévu une zone de rétention (ØR.8.Fig2) permettant de maintenir le filtre dans son logement mais aussi de le changer facilement dans le cas d'utilisation de bouchons jetables ou de le retirer temporairement dans le cas d'utilisation d'adaptateurs moulés sur mesure réutilisable après leur nettoyage.

Le perçage du bouchon (s.Fig1) allant vers le tympan devra être d'un diamètre supérieur ou égale à 2 mm afin d'éviter de produire des interactions acoustiques.

Un cordon attaché par collage ou mécaniquement pourra être utilisé pour relier les deux filtres.

## Revendications

1. Dispositif de protection anti-bruit apte à être utilisé dans un bouchon d'oreille, comprenant un système acoustique modulaire composé de l'assemblage d'une pièce d'entrée acoustique (2), d'une pièce de sortie acoustique (2) séparées par un empilement acoustique (M) et **caractérisé en ce qu'**il comporte un cône (6) en direction du tympan placé dans la pièce de sortie acoustique (2) ayant une forme fonction d'une courbe hyperbolique de type f(x) = 1/x², et un résonateur acoustique (5) dans la pièce de sortie acoustique (2) constitué par le volume intérieur du cylindre de la pièce de sortie acoustique (2) diminué du volume occupé par la forme intérieure du cône acoustique (6).

2. Dispositif selon la revendication 1, **caractérisé en ce que** ledit empilement acoustique (M) comprend au moins une membrane poreuse, un ou plusieurs joints d'étanchéité et une ou plusieurs grilles métalliques de protection.

3. Dispositif selon les revendications 1 et 2 **caractérisé en ce que** l'assemblage entre les pièces d'entrée acoustique (3) et sortie acoustique (2) est réalisé par collage ou clipsage en compressant l'empilement acoustique (M) afin d'obtenir son étanchéité sur son pourtour.

4. Dispositif selon la revendication 1 ou 2 **caractérisé en ce que** l'entrée (7) du cône acoustique (6) est concentrique avec l'empilement acoustique (M).

5. Dispositif selon la revendication 1 **caractérisé en ce qu'**il comporte une zone de rétention (8) permettant par clipsage le maintien et le retrait dudit dispositif dans le bouchon auriculaire (1).

6. Bouchon d'oreille étanche, **caractérisé en ce qu'**il comporte, intérieurement, un dispositif de protection anti-bruit selon l'une des revendications précédentes.

## Patentansprüche

1. Lärmschutzvorrichtung, welche geeignet ist, in einem Ohrstöpsel verwendet zu werden, welche ein modulares akustisches System aufweist, das aus dem Zusammenbau eines akustischen Eingangsteils (3) und eines akustischen Ausgangsteils (2) besteht, die durch einen akustischen Stapel (M) getrennt sind, **dadurch gekennzeichnet, dass** sie aufweist: einen Kegel (6) in Richtung des Trommelfells, der in dem akustischen Ausgangsteil (2) angeordnet ist und eine Form hat, die von einer hyperbolischen Kurve vom Typ f(x) = 1/x² abhängt, und einen akustischen Resonator (5) in dem akustischen Ausgangsteil (2), der aus dem Innenvolumen des Zylinders des akustischen Ausgangsteils (2), verringert um das von der Innenform des akustischen Kegels (6) eingenommene Volumen, besteht.

2. Vorrichtung nach Anspruch 1, **dadurch gekennzeichnet, dass** der akustische Stapel (M) mindestens eine poröse Membran, eine oder mehrere Dichtungen und ein oder mehrere metallische Schutzgitter aufweist.

3. Vorrichtung nach den Ansprüchen 1 und 2, **dadurch gekennzeichnet, dass** der Zusammenbau zwischen dem akustischen Eingangsteil (3) und dem akustischen Ausgangsteil (2) durch Kleben oder Klipsen unter Zusammendrücken des akustischen Stapels (M), um seine Dichtigkeit an seinem Umfang zu erzielen, durchgeführt wird.

4. Vorrichtung nach Anspruch 1 oder 2, **dadurch gekennzeichnet, dass** der Eingang (7) des akustischen Kegels (6) konzentrisch mit dem akustischen Stapel (M) ist.

5. Vorrichtung nach Anspruch 1, **dadurch gekennzeichnet, dass** sie einen Haltebereich (8) aufweist, der durch Klipsen das Halten und das Entfernen der Vorrichtung in dem bzw. aus dem Ohrstöpsel (1) ermöglicht.

6. Dichter Ohrstöpsel, **dadurch gekennzeichnet, dass** er innen eine Lärmschutzvorrichtung nach einem der vorhergehenden Ansprüche aufweist.

## Claims

1. Anti-noise protection device which can be used in an ear plug,
comprising a modular acoustic system assembled from an acoustic inlet part (3) and an acoustic output part (2), which are separated by an acoustic stack (M), and **characterized in that** it comprises a cone (6), oriented towards the eardrum and placed in the acoustic output part (2), whose shape is a function of a hyperbolic curve of the type f (x) = 1/x², and an acoustic resonator (5) in the acoustic output part (2), consisting of the internal volume of the cylinder of the acoustic output part (2) minus the volume occupied by the internal shape of the acoustic cone (6).

2. Device according to Claim 1, **characterized in that** said acoustic stack (M) comprises at least one porous membrane, one or more seals and one or more protective metal meshes.

3. Device according to Claims 1 and 2, **characterized in that** the acoustic inlet part (3) and the acoustic output part (2) are assembled by adhesive bonding or by snap-fitting, compressing the acoustic stack (M) such that it is sealed over its periphery.

4. Device according to Claim 1 or 2, **characterized in that** the inlet (7) of the acoustic cone (6) is concentric with the acoustic stack (M).

5. Device according to Claim 1, **characterized in that** it comprises a retaining region (8) allowing said device to be held in and removed from the ear plug (1) by snap-fitting.

6. Sealed ear plug, **characterized in that** it comprises, inside it, an anti-noise protection device according to one of the preceding claims.
